# EUROPEAN PATENT SPECIFICATION

(11) **EP 4 301 346 B1**
(45) Date of publication and mention of the grant of the patent: **19.08.2026**
(21) Application number: 22709764.9
(22) Date of filing: 02.03.2022
(51) Int. Cl.: A61K 9/20, A61K 31/155

(54) **STABLE PHARMACEUTICAL COMPOSITIONS OF METFORMIN**
STABILE PHARMAZEUTISCHE ZUSAMMENSETZUNG VON METFORMIN
COMPOSITION PHARMACEUTIQUE STABLE DE METFORMIN

(30) Priority: 03.03.2021 FI 20215229
(43) Date of publication of application: 10.01.2024
(60) Divisional application: 26191604.3
(73) Proprietor: ORION CORPORATION, 02200 Espoo (FI)
(72) Inventor: HAAJANTO, Tapio, 02101 Espoo (FI); PARTANEN, Marja, 02101 Espoo (FI); SALMIA, Jukka, 02101 Espoo (FI)
(74) Representative: J A Kemp LLP
(86) International application number: PCT/FI2022/050133
(87) International publication number: WO 2022/184981

(56) References cited:
- WO-A2-2007/031887
- WO-A2-2012/031124
- CN-A- 107 184 559
- US-A1- 2003 078 269
- US-A1- 2020 289 420
- ZMYSLOWSKI ADAM ET AL: "N-Nitrosodimethylamine Contamination in the Metformin Finished Products", MOLECULES, vol. 25, no. 22, 1 November 2020 (2020-11-01), DE, pages 5304, XP055801965, ISSN: 1433-1373, DOI: 10.3390/molecules25225304

## Description

### Technical field

The present invention relates to prolonged release tablet compositions, comprising metformin or a pharmaceutically acceptable salt thereof as a sole active ingredient. The compositions show improved chemical stability against formation of potentially carcinogenic nitrosamine compounds such as N-nitrosodimethylamine (NDMA).

### Background of the invention

Metformin is a commonly used anti-diabetic drug, which is the preferred initial medication for most of the patients with type 2 diabetes mellitus. Metformin has a relatively high allowed dosage up to 2-3 g/day in Europe and the USA. Recent detection of excess amounts of potentially carcinogenic nitrosamine impurities, particularly N-nitrosodimethylamine (NDMA), in metformin dosage forms, has created concern among health care providers and patients, and has caused recall of many metformin dosage forms, particularly extended release tablets. Recommended maximum allowed limit of NDMA in metformin products is set to 0.032 ppm by the European Medicines Agency. The root cause of NDMA source in metformin products has been investigated but definitive cause has remained unsolved.

Thus, there is a need for metformin compositions, which would avoid the problem of nitrosamine impurities in the dosage forms.

WO 2007/031887 A2 describes a pharmaceutical composition in the form of tablets, which constitute an orally administered, controlled drug delivery system that will release metformin or its pharmaceutically acceptable salt in a controllable manner.

### Summary of the invention

It has been found that the formation nitrosamines such as NDMA in metformin products can be reduced by introducing inhibitors of peroxide-induced oxidation such as citric acid or ascorbic acid into the metformin formulations. It was also found that peroxides are commonly present as impurities in excipient materials typically used in the preparation of metformin products. While not wishing to be bound by theory, it is believed that the formation of nitrosamines is caused by these peroxide impurities reacting with metformin during the manufacture and storage of the metformin products. The reactivity of peroxides can be reduced by inhibitors of peroxide-induced oxidation leading to reduced formation of nitrosamines such as NDMA in metformin products.

Thus, according to one embodiment of the invention, the present invention provides the use of an inhibitor of peroxide-induced oxidation for reducing the formation of nitrosamines, particularly NDMA, in a prolonged release tablet composition, which comprises metformin or a pharmaceutically acceptable salt thereof as a sole active ingredient and at least one excipient, wherein the inhibitor of peroxide-induced oxidation is an antioxidant or a stabilizing acid.

According to another embodiment, the present invention provides a prolonged release tablet composition comprising metformin or a pharmaceutically acceptable salt thereof as a sole active ingredient, at least one excipient and from about 0.01 to about 10 %, preferably from about 0.05 to about 5 %, typically from about 0.1 to about 2 %, for example from about 0.25 to about 1 %, of an inhibitor of peroxide-induced oxidation, per total weight of excipients, wherein the inhibitor of peroxide-induced oxidation is an antioxidant or a stabilizing acid, and wherein the composition is not an effervescent or a gastric floating composition which comprises an acid together with a base for the release of carbon dioxide.

According to another embodiment, the present invention provides a method for preparing a prolonged release tablet composition comprising metformin or a pharmaceutically acceptable salt thereof as a sole active ingredient and at least one excipient, wherein the composition is not an effervescent or a gastric floating composition which comprises an acid together with a base for the release of carbon dioxide, which method comprises contacting the at least one excipient with an amount of an inhibitor of peroxide-induced oxidation effective to reduce the formation nitrosamines in the composition, wherein the inhibitor of peroxide-induced oxidation is an antioxidant or a stabilizing acid.

### Detailed description of the invention

The term "ppm", as used herein, refers to parts per million per weight. Thus, 1 ppm per weight corresponds to 1 mg/kg.

The term "inhibitor of peroxide-induced oxidation", as used herein, refers to a group of substances that are capable of inhibiting peroxide-induced degradation reactions of metformin. The inhibition can take place, for example, by eliminating or reducing peroxides or by inhibiting, preventing or reducing oxidative reactions of peroxides. Inhibitors of peroxide-induced oxidation include "antioxidants" and "stabilizing acids", as referred to below.

The term "antioxidant", as used herein, refers to a group of substances that are capable of inhibiting, preventing or reducing oxidative reactions. Preferred are water-soluble antioxidants, examples of which include ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) or pharmaceutically acceptable salts thereof.

The term "stabilizing acid", as used herein, refers to a pharmaceutically acceptable acid having pKa lower than 4.7, for example from about -8 to lower than 4.7, from about -2 to about 4.5, or from about 1 to about 4.5, and being capable of stabilizing metformin against peroxide induced reactions in pharmaceutical compositions, particularly tablets. Examples of stabilizing acids include citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid.

The term "nitrosamine", as used herein, refers to an organic compound of the chemical structure R₁(R₂)N-N=O, wherein R₁ and R₂ is an alkyl or a derivative of an alkyl group. Examples of "nitrosamine" include *N*-nitrosodimethylamine (NDMA), *N*-nitrosodiethylamine (NDEA) and *N*-nitroso-N-methyl-4-aminobutyric acid (NMBA). Nitrosamine impurities may increase the risk of cancer in people exposed to them above acceptable levels and over long periods.

The term "cellulose derivative", as used herein, refers to cellulose ether derivatives commonly used as excipients in pharmaceutical dosage forms such as tablets. Examples of "cellulose derivatives" include methylcellulose (MC), ethyl cellulose (EC), hydroxyethyl cellulose (HEC), hydroxypropyl cellulose (HPC), hydroxypropylmethyl cellulose (HPMC), carboxymethyl cellulose (CMC) and sodium carboxymethyl cellulose (NaCMC).

The term "methacrylate polymer", as used herein, refers to a polymer, usually a copolymer, prepared from a methacrylate or methyl methacrylate monomer. Examples of methacrylate polymers include EUDRAGIT^{®} L100-55 which is a copolymer of methacrylic acid and ethyl acrylate and EUDRAGIT^{®} L100 which is a copolymer of methacrylic acid and methyl methacrylate and EUDRAGIT^{®} RS 100 which is a copolymer of ethyl acrylate, methyl methacrylate and a low content of methacrylic acid ester with quaternary ammonium groups.

The term "polyvinylpyrrolidone derivative", as used herein, refers to polyvinylpyrrolidone (also called povidone or PVP), copolymers of 1-vinyl-2-pyrrolidone such as copovidone (copolymer of 1-vinyl-2-pyrrolidone and vinyl acetate) and cross-linked polyvinylpyrrolidone (crospovidone).

The term "polysorbate", as used herein, refers to oleate esters of sorbitol and its anhydrides, typically copolymerized with ethylene oxide. Preferred polysorbates are Polysorbate 20 (poly(ethylene oxide) (20) sorbitan monolaurate, Tween 20) or Polysorbate 80 (poly(ethylene oxide) (80) sorbitan monolaurate, Tween 80).

The term "polyethylene glycol", as used herein, refers to a polymer containing ethylene glycol monomer units of formula -O-CH₂-CH₂-. Polyethylene glycols (also known as Macrogols) may have average molecular weight from about 200 to about 9000 g/mol, from about 200 to about 5000 g/mol or from about 200 to about 900 g/mol. Examples of polyethylene glycol include polyethylene glycols include PEG 200, PEG 300, PEG 400 and PEG 1000.

The term "water soluble", as used herein, refers to water solubility of 10 mg/ ml or higher, preferably 20 mg/ml or higher, more preferably 50 mg/ml or higher.

The present invention relates to the use of an inhibitor of peroxide-induced oxidation for reducing the formation nitrosamines, particularly NDMA, in a prolonged release tablet composition which comprises metformin or a pharmaceutically acceptable salt thereof as a sole active ingredient and at least one excipient, wherein the inhibitor of peroxide-induced oxidation is an antioxidant or a stabilizing acid.

Particularly suitable antioxidants are water-soluble antioxidants such as ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) and pharmaceutically acceptable salts thereof. Preferred are ascorbic acid, sulfites, bisulfites and metabisulfites and pharmaceutically acceptable salts thereof, for example sodium salts such as sodium ascorbate, sodium sulfite, sodium bisulfite and sodium metabisulfite. Particularly preferred is ascorbic acid.

Suitable stabilizing acids are pharmaceutically acceptable acids having pKa lower than 4.7, for example from about -8 to lower than 4.7, from about -2 to about 4.5, or from about 1 to about 4.5. Particularly suitable stabilizing acids include, for example, citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid. Preferred are citric acid, tartaric acid, phosphoric acid, malic acid and malonic acid. Citric acid is particularly preferred as it is edible component and not considered active ingredient in oral pharmaceutical products.

Inhibitors of peroxide-induced oxidation are suitably used in metformin prolonged release tablets, which comprise pharmaceutical excipients containing peroxide impurities. Typically, in humans a prolonged release tablet of metformin hydrochloride exhibits Tₘₐₓ (the time to reach maximum concentration in plasma) at the time which is at least about 4 hours, more typically at least about 5 hours, for example about 6-7 hours, after ingestion.

Pharmaceutical excipients which are particularly used in tablets and which have been found to contain peroxides include, but are not limited to, cellulose derivatives, methacrylate polymers, polyvinylpyrrolidone derivatives, polysorbates and polyethylene glycols.

Typical cellulose derivatives used in tablets include, but are not limited to, methylcellulose (MC), ethyl cellulose (EC), hydroxyethyl cellulose (HEC), hydroxypropyl cellulose (HPC), hydroxypropylmethyl cellulose (HPMC), carboxymethyl cellulose (CMC) and sodium carboxymethyl cellulose (NaCMC). HPMC and HPC are particular examples of cellulose derivatives which commonly contains peroxides.

Typical methacrylate polymers used in tablets include, but are not limited to, a copolymer of methacrylic acid and ethyl acrylate (available e.g. under the trade name EUDRAGIT^{®} L100-55), a copolymer of methacrylic acid and ethyl acrylate (available e.g. under the trade name EUDRAGIT^{®} L100), and a copolymer of ethyl acrylate, methyl methacrylate and a low content of methacrylic acid ester with quaternary ammonium groups (available e.g. under the trade name EUDRAGIT^{®} RS 100).

Typical polyvinylpyrrolidone derivatives used in tablets include, but are not limited to, polyvinylpyrrolidone (PVP, also known as povidone), copovidone (copolymer of 1-vinyl-2-pyrrolidone and vinyl acetate) and cross-linked polyvinylpyrrolidone (crospovidone). PVP is a particular example of a polyvinylpyrrolidone derivative which commonly contains peroxides.

Typical polysorbates used in tablets include, but are not limited to, Polysorbate 20 (poly(ethylene oxide) (20) sorbitan monolaurate) and Polysorbate 80 (poly(ethylene oxide) (80) sorbitan monolaurate, also known as Tween 80).

Typical polyethylene glycols (also known as Macrogols) used in tablets include, but are not limited to, PEG 200, PEG 300, PEG 400, PEG 1000 and PEG 6000.

According to one aspect, the present invention discloses a prolonged release tablet composition comprising metformin or a pharmaceutically acceptable salt thereof as a sole active ingredient, at least one excipient and from about 0.01 to about 10 %, preferably from about 0.05 to about 5 %, typically from about 0.1 to about 2 %, for example from about 0.25 to about 1 %, of an inhibitor of peroxide-induced oxidation, per total weight of excipients, wherein the inhibitor of peroxide-induced oxidation is an antioxidant or a stabilizing acid, and wherein the composition is not an effervescent or a gastric floating composition which comprises an acid together with a base for the release of carbon dioxide.

Inhibitors of peroxide-induced oxidation to be used in the above pharmaceutical composition are antioxidants, particularly water-soluble antioxidants, and stabilizing acids. Suitable water-soluble antioxidants include ascorbic acid, acetyl cysteine, cysteine, thiourea, 1-thiosorbitol, sulphites (e.g. sodium sulfite), bisulfites (e.g. sodium bisulfite), metabisulfites (e.g. sodium metabisulfite), thiosulfates (e.g. sodium thiosulfate) or pharmaceutically acceptable salts thereof. Preferred are ascorbic acid, sulfites, bisulfites and metabisulfites and pharmaceutically acceptable salts thereof, for example sodium salts such as sodium ascorbate, sodium sulfite, sodium bisulfite and sodium metabisulfite. Ascorbic acid is particularly preferred. Examples of suitable stabilizing acids include citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid and hydrochloric acid. Preferred are citric acid, tartaric acid, phosphoric acid, malic acid and malonic acid. Citric acid being particularly preferred. In one aspect, the inhibitor of peroxide-induced oxidation is citric acid or ascorbic acid.

According to one embodiment, the present invention discloses a prolonged release tablet composition comprising
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 5 - 60 %, preferably, 10 - 50 %, for example 20 - 40 %, per weight of the composition, of at least one excipient.

According to one aspect of the above embodiment, the excipient comprises at least one excipient selected from the group consisting of cellulose derivatives, methacrylate polymers, polyvinylpyrrolidone derivatives, polysorbates and polyethylene glycols. According to another aspect, the excipient comprises at least one excipient selected from the group consisting of cellulose derivatives, methacrylate polymers and polyvinylpyrrolidone derivatives. According to another aspect, the excipient comprises at least one excipient selected from the group consisting of HPMC, HPC, povidone and copovidone. According to another aspect, the excipient comprises at least one excipient selected from the group consisting of HPMC, povidone and copovidone. According to another aspect, the excipient comprises at least one excipient selected from the group consisting of HPMC and copovidone. According to another aspect, the excipient is HPMC.

The inhibitor of peroxide-induced oxidation is an antioxidant, particularly a water-soluble antioxidant, or a stabilizing acid. According to one aspect, the water-soluble antioxidant is ascorbic acid, a sulfite, a bisulfite, a metabisulfite or a pharmaceutically acceptable salt thereof, for example, a sodium salt such as sodium ascorbate, sodium sulfite, sodium bisulfite or sodium metabisulfite. In one aspect, the antioxidant is ascorbic acid.

According to another aspect, the stabilizing acid in the above aspect is citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid. In one aspect, the stabilizing acid is citric acid, tartaric acid, phosphoric acid, malic acid or malonic acid. In one aspect, the stabilizing acid is citric acid.

In still another aspect, the inhibitor of peroxide-induced oxidation is citric acid or ascorbic acid.

According to still another embodiment, the present invention discloses a prolonged release tablet composition comprising
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 0.5 - 10 %, preferably 1 - 8 %, for example 2 - 5 %, per weight of the composition, of a binder;
(d) 5 - 50 %, preferably 10 - 45 %, for example 15 - 40 %, per weight of the composition, of a release controlling polymer selected from cellulose derivatives and methacrylate polymers; and
(e) 0.1 - 5 %, preferably 0.2 - 2 %, for example 0.3 - 1 %, per weight of the composition, of lubricant selected from magnesium stearate and stearic acid.

According to one aspect of the above embodiment, the inhibitor of peroxide-induced oxidation is a water-soluble antioxidant or a stabilizing acid. According to another aspect, the water-soluble antioxidant is ascorbic acid, a sulfite, a bisulfite, a metabisulfite or a pharmaceutically acceptable salt thereof, for example a sodium salt such as sodium ascorbate, sodium sulfite, sodium bisulfite or sodium metabisulfite. In one aspect, the antioxidant is ascorbic acid.

According to another aspect, the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid. In one aspect, the stabilizing acid is citric acid, tartaric acid, phosphoric acid, malic acid or malonic acid. In one aspect, the stabilizing acid is citric acid. In still another aspect, the inhibitor of peroxide-induced oxidation is citric acid or ascorbic acid. In one aspect, the binder is povidone (PVP) or copovidone. In one aspect, the binder is povidone (PVP). In another aspect, the binder is copovidone. In one aspect, the release-controlling polymer is HPMC or HPC. In one aspect, the release-controlling polymer is HPMC. In another aspect, the release-controlling polymer is HPC.

The composition according to the present invention is in the form of a tablet intended for prolonged release of the active ingredient. The composition is in the form of a prolonged release tablet. Typically, in humans a prolonged release tablet of metformin hydrochloride exhibits Tₘₐₓ (the time to reach maximum concentration in plasma) at the time which is at least about 4 hours, more typically at least about 5 hours, for example about 6-7 hours, after ingestion.

The composition according to the invention comprises metformin or a pharmaceutically acceptable salt thereof as a sole active ingredient.

The composition according to the invention is not an effervescent or a gastric floating composition, particularly a composition which comprises an acid, for example citric acid, together with a base, for example NaHCO₃, for the release of carbon dioxide.

The preparation of the composition comprises contacting at least one excipient with an amount of an inhibitor of peroxide-induced oxidation effective to reduce the formation nitrosamines in the composition.

The prolonged release tablet composition can be prepared by known methods, for example by wet granulation, dry granulation or dry compression. According to one preferred aspect of the invention, the prolonged release tablet composition is prepared by wet granulation comprising water as granulating liquid.

The method of preparing the prolonged release tablet by wet granulation generally comprises the steps of
(a) dissolving the inhibitor of peroxide-induced oxidation in a granulating liquid comprising water;
(b) granulating the mixture comprising at least one excipient with the granulating liquid of step (a);
(c) drying the wet granules;
(d) optionally mixing the granules with further excipients; and
(e) compressing the resulting mass into tablets; and, optionally, coating the tablet with one or further pharmaceutically acceptable film-coating agent.

The mixture of step (b) may also comprise metformin or a pharmaceutically acceptable salt thereof. Alternatively, step (c) can be followed by a further step of mixing metformin or a pharmaceutically acceptable salt thereof with the granules obtained in step (c) followed by further granulation of the resulting mixture with a granulating liquid comprising water; and drying the resulting granules.

The wet granulated prolonged release tablet is suitably prepared, for example, by first mixing metformin or a pharmaceutically acceptable salt thereof, the binder (for example povidone or copovidone) and a release controlling polymer (for example HPMC or HPC), optionally with other excipients, in a suitable granulator vessel, for example high shear granulator. The inhibitor of peroxide-induced oxidation (for example citric acid or ascorbic acid) is then dissolved in granulating liquid, preferably water, for obtaining a granulating liquid. The above mixture is then granulated by spaying the granulating liquid in a suitable vessel, for example a fluidized bed granulator. The obtained granules are dried and screened, for example, using a screening mill unit, optionally together with further excipients (for example a glidant such as fumed silica). Lubricant (for example magnesium stearate or stearic acid) can then be added to the mass of the previous step followed by blending. The tablet mass can then be compressed into tablet cores, for example, in a power assisted rotary tablet press.

The tablet cores can be provided with a water-soluble film coating, if desired, to facilitate tablet swallowing, to protect from direct contact with the drug substance and to improve aesthetics. Suitable film coating agents can be selected from the group of plasticizers, film-forming agents and colorants. Optionally an anti-tacking agent or opacifier can be used. The plasticizer, such as polyethylene glycol (PEG), the film-forming agent, such as hydroxypropylmethyl cellulose (HPMC), and the colorants, such as ferric oxide and titanium dioxide, are combined with film-coating liquids, preferably water, to result in a homogeneous coating suspension which is brought up, preferably sprayed, on the tablets in a suitable coating device, such as for example a perforated drum coater.

Metformin or a pharmaceutically acceptable salt thereof, particularly hydrochloride salt, is suitably administered in an amount ranging from about 500 mg to about 3000 mg, per day to the patient, for the treatment of type 2 diabetes mellitus. The dose can be administered once daily or divided to several times a day, for example twice daily. The prolonged release tablet composition of the invention, may comprise metformin or a pharmaceutically acceptable salt thereof, particularly hydrochloride salt, in an amount ranging from about 200 mg to about 2000 mg, typically from about 500 mg to about 1000 mg, for example 500 mg, 750 mg or 1000 mg of metformin hydrochloride. Such composition can be administered once or several times a day, for example one tablet or several tablets once, twice or several times daily.

The invention is further illustrated by the following examples, which are not meant to limit the scope of the invention.

### Example 1. Preparation of prolonged release metformin HCl tablets

Metformin prolonged release tablet formulations A - E were prepared. Formulations A and E were prepared by dry mixing metformin HCl, hydroxypropyl methylcellulose (HPMC K4M) and copovidone or low substituted hydroxypropylcellulose (L-HPC LH-21) followed by wet granulation of the mixture by spraying water to the mixture. The formed granules were dried. The dry granules were mixed with fumed silica (Aerosil 200) and magnesium stearate to produce a tablet mass which was finally compressed into tablets. Formulations B-D were prepared similarly except that the mixture of HPMC and copovidone was first treated by spraying the mixture with a water solution (10 %) of citric and/or ascorbic acid followed by drying the mixture. The final formulations of each tablets mass A-E are shown in Table 1. From each tablet mass was compressed tablets with total weight of about 460 mg.

**Table 1. Tested formulations**

| **Material** | **A (%)** | **B (%)** | **C (%)** | **D (%)** | **E (%)** |
|---|---|---|---|---|---|
| **Intragranular part** | | | | | |
| Metformin HCl | 65.8 | 64.7 | 64.7 | 64.8 | 65.8 |
| HPMC K4M | 28.9 | 28.5 | 28.5 | 28.5 | 28.9 |
| Copovidone | 2.9 | 2.8 | 2.8 | 2.8 | - |
| Citric acid | - | 1.6 | - | 0.9 | - |
| Ascorbic acid | - | - | 1.6 | 0.6 | - |
| L-HPC LH-21 | - | - | - | - | 2.9 |

| **Extragranular part** | | | | | |
|---|---|---|---|---|---|
| Fumed silica (Aerosil 200) | 1.9 | 1.9 | 1.9 | 1.9 | 1.9 |
| Magnesium stearate | 0.5 | 0.5 | 0.5 | 0.5 | 0.5 |
| **Total** | 100.0 | 100.0 | 100.0 | 100.0 | 100.0 |

### Example 2. Stability of the extended release metformin HCl tablets against NDMA formation

The tablet mass and the compressed tablets of Example 1 were put to the stressed stability test for 4 days at 60 °C. The initial amount of NDMA in the tablet mass and compressed tablets and after stressing for 4 days were determined. The results are shown in Tables 2 and 3. In the Tables, 0 ppm means NDMA level under the detection limit (0.003 ppm). The maximum allowed limit for NDMA in metformin products currently set by the European Medicines Agency is 0.032 ppm.

**Table 2. Formation of NDMA in the tablet mass**

| | **Formulation** | **NDMA on Day 0 (ppm)** | **NDMA on Day 4 (ppm)** |
|---|---|---|---|
| **Tablet mass** | A | 0.022 | 0.075 |
| | B | 0 | 0 |
| | C | 0 | 0 |
| | D | 0 | 0 |
| | E | 0.025 | 0.092 |

**Table 3. Formation of NDMA in the pressed tablets**

| | **Formulation** | **NDMA on Day 0 (ppm)** | **NDMA on Day 4 (ppm)** |
|---|---|---|---|
| **Tablet** | A | 0.019 | 0.059 |
| | B | 0.016 | 0 |
| | C | 0 | 0 |
| | D | 0 | 0 |
| | E | 0.019 | 0.086 |

It can be seen that using 5 % of citric or ascorbic acid, per total weight of the granule excipients, results in significantly reduced NDMA formation during manufacture of the tablet mass and also during the storage of the tablets in the stressed conditions. The experiment was repeated using 0.5 %, 1.5 % and 3 % of citric or ascorbic acid, per total weight of the granule excipients and similar reduction of NDMA formation was observed.

## Claims

1. Use of an inhibitor of peroxide-induced oxidation for reducing the formation of nitrosamines in a prolonged release tablet composition which comprises metformin or a pharmaceutically acceptable salt thereof as a sole active ingredient and at least one excipient, wherein the inhibitor of peroxide-induced oxidation is an antioxidant or a stabilizing acid.

2. Use according to claim 1, wherein
(i) the antioxidant is a water-soluble antioxidant, optionally wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof, and/or
(ii) the stabilizing acid is citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid or hydrochloric acid.

3. Use according to claim 1, wherein the inhibitor of peroxide-induced oxidation is citric acid or ascorbic acid.

4. Use according to any one of claims 1 to 3, wherein the excipient is selected from the group consisting of cellulose derivatives, methacrylate polymers, polyvinylpyrrolidone derivatives, polysorbates and polyethylene glycols.

5. A prolonged release tablet composition comprising metformin or a pharmaceutically acceptable salt thereof as a sole active ingredient, at least one excipient and from about 0.01 to about 10 %, preferably from about 0.05 to about 5 %, more preferably from about 0.1 to about 2 %, of an inhibitor of peroxide-induced oxidation, per total weight of excipients, wherein the inhibitor of peroxide-induced oxidation is an antioxidant or a stabilizing acid, and wherein the composition is not an effervescent or a gastric floating composition which comprises an acid together with a base for the release of carbon dioxide.

6. A composition according to claim 5, wherein
(i) the antioxidant is a water-soluble antioxidant, optionally wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof, and/or
(ii) the stabilizing acid is citric acid, tartaric acid, phosphoric acid, malic acid, lactic acid, methanesulfonic acid, ethanesulfonic acid, *p*-toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid.

7. A composition according to claim 5, wherein the inhibitor of peroxide-induced oxidation is citric acid or ascorbic acid.

8. A composition according to any one of claims 5 to 7, wherein the excipient is selected from the group consisting of cellulose derivatives, methacrylate polymers, polyvinylpyrrolidone derivatives, polysorbates and polyethylene glycols.

9. A composition according to any of claims 5 to 8 comprising
(i)
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 5 - 60 %, preferably, 10 - 50 %, for example 20 - 40 %, per weight of the composition, of at least one excipient, or
(ii)
(a) 40 - 90 %, preferably 50 to 80 %, for example 55 - 75 %, per weight of the composition, of metformin or a pharmaceutically acceptable salt thereof,
(b) 0.01 - 5 %, preferably 0.02 - 2 %, for example 0.05 - 1 %, for example 0.1 - 0.5 %, per weight of the composition, of an inhibitor of peroxide-induced oxidation;
(c) 0.5 - 10 %, preferably 1 - 8 %, for example 2 - 5 %, per weight of the composition, of a binder;
(d) 5 - 50 %, preferably 10 - 45 %, for example 15 - 40 %, per weight of the composition, of a release controlling polymer selected from cellulose derivatives and methacrylate polymers; and
(e) 0.1 - 5 %, preferably 0.2 - 2 %, for example 0.3 - 1 %, per weight of the composition, of lubricant selected from magnesium stearate and stearic acid.

10. A method for preparing a prolonged release tablet composition comprising metformin or a pharmaceutically acceptable salt thereof as a sole active ingredient and at least one excipient, wherein the composition is not an effervescent or a gastric floating composition which comprises an acid together with a base for the release of carbon dioxide, which method comprises contacting the at least one excipient with an amount of an inhibitor of peroxide-induced oxidation effective to reduce the formation nitrosamines in the composition, wherein the inhibitor of peroxide-induced oxidation is an antioxidant or a stabilizing acid.

11. A method according to claim 10, wherein
(i) the antioxidant is a water-soluble antioxidant, optionally wherein the water-soluble antioxidant is ascorbic acid, acetyl cysteine, cysteine, thiourea, 1-thiosorbitol, a sulfite, a bisulfite, a metabisulfite, a thiosulfate or a pharmaceutically acceptable salt thereof, and/or
(ii) the stabilizing acid is citric acid, tartaric acid, phosphoric acid, malic acid, lactic acid, methanesulfonic acid, ethanesulfonic acid, p-toluenesulfonic acid, oxalic acid, malonic acid, succinic acid, glutaric acid, adipic acid, gluconic acid, glucuronic acid, glutamic acid, fumaric acid, maleic acid, hyaluronic acid or a hydrochloric acid.

12. A method according to claim 10, wherein the inhibitor of peroxide-induced oxidation is citric acid or ascorbic acid.

13. A method according to any one of claims 10 to 12, wherein the excipient is selected from the group consisting of cellulose derivatives, methacrylate polymers, polyvinylpyrrolidone derivatives, polysorbates and polyethylene glycols.

14. A method according to any of claims 10 to 13, comprising the steps of
(a) dissolving the inhibitor of peroxide-induced oxidation in a granulating liquid comprising water;
(b) granulating the mixture comprising at least one excipient with the granulating liquid of step (a);
(c) drying the wet granules;
(d) optionally mixing the granules with further excipients; and
(d) compressing the resulting mass into tablets; and, optionally, coating the tablet with one or further pharmaceutically acceptable film-coating agent.

15. A method according to claim 14, wherein the mixture of step (b) also comprises metformin or a pharmaceutically acceptable salt thereof, optionally wherein step (c) is followed by a further step of mixing metformin or a pharmaceutically acceptable salt thereof with the granules obtained in step (c) followed by further granulation of the resulting mixture with a granulating liquid comprising water; and drying the resulting granules.

## Patentansprüche

1. Verwendung eines Inhibitors der peroxidinduzierten Oxidation zur Verringerung der Bildung von Nitrosaminen in einer Tablettenzusammensetzung mit verzögerter Freisetzung, die Metformin oder ein pharmazeutisch annehmbares Salz davon als einzigen Wirkstoff und mindestens einen Hilfsstoff umfasst, wobei der Inhibitor der peroxidinduzierten Oxidation ein Antioxidans oder eine stabilisierende Säure ist.

2. Verwendung nach Anspruch 1, wobei
(i) das Antioxidans ein wasserlösliches Antioxidans ist, optional wobei das wasserlösliche Antioxidans Ascorbinsäure, Acetylcystein, Cystein, Thioharnstoff, 1-Thiosorbit, ein Sulfit, ein Bisulfit, ein Metabisulfit, ein Thiosulfat oder ein pharmazeutisch annehmbares Salz davon ist, und/oder
(ii) die stabilisierende Säure Zitronensäure, Weinsäure, Phosphorsäure, Apfelsäure, Milchsäure, Methansulfonsäure, Ethansulfonsäure, p-Toluolsulfonsäure, Oxalsäure, Malonsäure, Bernsteinsäure, Glutarsäure, Adipinsäure, Gluconsäure, Glucuronsäure, Glutaminsäure, Fumarsäure, Maleinsäure, Hyaluronsäure oder Salzsäure ist.

3. Verwendung nach Anspruch 1, wobei der Inhibitor der peroxidinduzierten Oxidation Zitronensäure oder Ascorbinsäure ist.

4. Verwendung nach Anspruch 1 bis 3, wobei der Hilfsstoff aus der Gruppe ausgewählt ist, die aus Cellulosederivaten, Methacrylatpolymeren, Polyvinylpyrrolidon-Derivaten, Polysorbaten und Polyethylenglykolen besteht.

5. Tablettenzusammensetzung mit verzögerter Freisetzung, die Folgendes umfasst: Metformin oder ein pharmazeutisch annehmbares Salz davon als einzigen Wirkstoff, mindestens einen Hilfsstoff und etwa 0,01 bis etwa 10 %, vorzugsweise etwa 0,05 bis etwa 5 %, noch bevorzugter etwa 0,1 bis etwa 2 %, eines Inhibitors der peroxidinduzierten Oxidation, bezogen auf das Gesamtgewicht der Hilfsstoffe, wobei der Inhibitor der peroxidinduzierten Oxidation ein Antioxidans oder eine stabilisierende Säure ist. wobei die Zusammensetzung keine Brause- oder im Magen schwimmende Zusammensetzung umfasst, die eine Säure zusammen mit einer Basis zur Freisetzung von Kohlendioxid umfasst.

6. Zusammensetzung nach Anspruch 5, wobei
(i) das Antioxidans ein wasserlösliches Antioxidans ist, optional wobei das wasserlösliche Antioxidans Ascorbinsäure, Acetylcystein, Cystein, Thioharnstoff, 1-Thiosorbit, ein Sulfit, ein Bisulfit, ein Metabisulfit, ein Thiosulfat oder ein pharmazeutisch annehmbares Salz davon ist, und/oder
(ii) die stabilisierende Säure Zitronensäure, Weinsäure, Phosphorsäure, Apfelsäure, Milchsäure, Methansulfonsäure, Ethansulfonsäure, p-Toluolsulfonsäure, Oxalsäure, Malonsäure, Bernsteinsäure, Glutarsäure, Adipinsäure, Gluconsäure, Glucuronsäure, Glutaminsäure, Fumarsäure, Maleinsäure, Hyaluronsäure oder Salzsäure ist.

7. Zusammensetzung nach Anspruch 5, wobei der Inhibitor der peroxidinduzierten Oxidation Zitronensäure oder Ascorbinsäure ist.

8. Zusammensetzung nach einem der Ansprüche 5 bis 7, wobei der Hilfsstoff aus der Gruppe ausgewählt ist, die aus Cellulosederivaten, Methacrylatpolymeren, Polyvinylpyrrolidon-Derivaten, Polysorbaten und Polyethylenglykolen besteht.

9. Zusammensetzung nach einem der Ansprüche 5 bis 8, die umfasst:
(i)
(a) 40-90 Gew.-%, vorzugsweise 50 bis 80 Gew.-%, beispielsweise 55-75 Gew.-%, bezogen auf das Gewicht der Zusammensetzung, an Metformin oder einem pharmazeutisch annehmbaren Salz davon,
(b) 0,01-5 %, vorzugsweise 0,02-2 %, beispielsweise 0,05-1 %, beispielsweise 0,1-0,5 %, bezogen auf das Gewicht der Zusammensetzung, eines Inhibitors der peroxidinduzierten Oxidation;
(c) 5-60 %, vorzugsweise 10-50 %, beispielsweise 20-40 %, bezogen auf das Gewicht der Zusammensetzung, mindestens eines Hilfsstoffs, oder
(ii)
(a) 40-90 Gew.-%, vorzugsweise 50 bis 80 Gew.-%, beispielsweise 55-75 Gew.-%, bezogen auf das Gewicht der Zusammensetzung, an Metformin oder einem pharmazeutisch annehmbaren Salz davon,
(b) 0,01-5 %, vorzugsweise 0,02-2 %, beispielsweise 0,05-1 %, beispielsweise 0,1-0,5 %, bezogen auf das Gewicht der Zusammensetzung, eines Inhibitors der peroxidinduzierten Oxidation;
(c) 0,5-10 %, vorzugsweise 1-8 %, beispielsweise 2-5 %, bezogen auf das Gewicht der Zusammensetzung, eines Bindemittels;
(d) 5-50 %, vorzugsweise 10-45 %, beispielsweise 15-40 %, bezogen auf das Gewicht der Zusammensetzung, eines die Freisetzung steuernden Polymers, ausgewählt aus Cellulosederivaten und Methacrylatpolymeren; und
(e) 0,1-5 %, vorzugsweise 0,2-2 %, beispielsweise 0,3-1 %, bezogen auf das Gewicht der Zusammensetzung, eines Gleitmittels, ausgewählt aus Magnesiumstearat und Stearinsäure.

10. Verfahren zur Herstellung einer Tablettenzusammensetzung mit verzögerter Freisetzung, die Metformin oder ein pharmazeutisch annehmbares Salz davon als einzigen Wirkstoff und mindestens einen Hilfsstoff umfasst, wobei die Zusammensetzung keine Brause- oder im Magen schwimmende Zusammensetzung umfasst, die eine Säure zusammen mit einer Basis zur Freisetzung von Kohlendioxid umfasst, wobei das Verfahren das Inkontaktbringen des mindestens einen Hilfsstoffs mit einer Menge eines Inhibitors der peroxidinduzierten Oxidation umfasst, die wirksam ist, um die Bildung von Nitrosaminen in der Zusammensetzung zu verringern, wobei der Inhibitor der peroxidinduzierten Oxidation ein Antioxidans oder eine stabilisierende Säure ist.

11. Verfahren nach Anspruch 10, wobei
(i) das Antioxidans ein wasserlösliches Antioxidans ist, optional wobei das wasserlösliche Antioxidans Ascorbinsäure, Acetylcystein, Cystein, Thioharnstoff, 1-Thiosorbit, ein Sulfit, ein Bisulfit, ein Metabisulfit, ein Thiosulfat oder ein pharmazeutisch annehmbares Salz davon ist, und/oder
(ii) die stabilisierende Säure Zitronensäure, Weinsäure, Phosphorsäure, Apfelsäure, Milchsäure, Methansulfonsäure, Ethansulfonsäure, p-Toluolsulfonsäure, Oxalsäure, Malonsäure, Bernsteinsäure, Glutarsäure, Adipinsäure, Gluconsäure, Glucuronsäure, Glutaminsäure, Fumarsäure, Maleinsäure, Hyaluronsäure oder Salzsäure ist.

12. Verfahren nach Anspruch 10, wobei der Inhibitor der peroxidinduzierten Oxidation Zitronensäure oder Ascorbinsäure ist.

13. Verfahren nach einem der Ansprüche 10 bis 12, wobei der Hilfsstoff aus der Gruppe ausgewählt ist, die aus Cellulosederivaten, Methacrylatpolymeren, Polyvinylpyrrolidon-Derivaten, Polysorbaten und Polyethylenglykolen besteht.

14. Verfahren nach einem der Ansprüche 10 bis 13, das folgende Schritte umfasst:
(a) Auflösen des Inhibitors der peroxidinduzierten Oxidation in einer Granulierflüssigkeit, die Wasser umfasst;
(b) Granulieren des Gemisches, das mindestens einen Hilfsstoff umfasst, mit der Granulierflüssigkeit aus Schritt (a);
(c) Trocknen der feuchten Granulate;
(d) optional Mischen der Granulate mit weiteren Hilfsstoffen; und
(e) Verpressen der resultierenden Masse zu Tabletten; und optional Beschichten der Tablette mit einem oder mehreren pharmazeutisch annehmbaren Filmüberzugsmitteln.

15. Verfahren nach Anspruch 14, wobei das Gemisch aus Schritt (b) auch Metformin oder ein pharmazeutisch annehmbares Salz davon umfasst, optional wobei auf Schritt (c) ein weiterer Schritt folgt, bei dem Metformin oder ein pharmazeutisch annehmbares Salz davon mit den in Schritt (c) erhaltenen Granulaten gemischt wird, gefolgt von einer weiteren Granulierung des resultierenden Gemisches mit einer Granulierflüssigkeit, die Wasser umfasst; und Trocknen der resultierenden Granulate.

## Revendications

1. Utilisation d'un inhibiteur d'oxydation induite par peroxyde destiné à réduire la formation de nitrosamines dans une composition de comprimé à libération prolongée qui comprend de la metformine ou un sel pharmaceutiquement acceptable de celle-ci en tant que principe actif unique et au moins un excipient, dans laquelle l'inhibiteur d'oxydation induite par peroxyde est un antioxydant ou un acide stabilisant.

2. Utilisation selon la revendication 1, dans laquelle
(i) l'antioxydant est un antioxydant hydrosoluble, éventuellement dans laquelle l'antioxydant hydrosoluble est l'acide ascorbique, l'acétyl cystéine, la cystéine, la thiourée, le 1-thiosorbitol, un sulfite, un bisulfite, un métabisulfite, un thiosulfate ou un sel pharmaceutiquement acceptable de ceux-ci, et/ou
(ii) l'acide stabilisant est l'acide citrique, l'acide tartrique, l'acide phosphorique, l'acide malique, l'acide lactique, l'acide méthanesulfonique, l'acide éthanesulfonique, l'acide *p-*toluènesulfonique, l'acide oxalique, l'acide malonique, l'acide succinique, l'acide glutarique, l'acide adipique, l'acide gluconique, l'acide glucuronique, l'acide glutamique, l'acide fumarique, l'acide maléique, l'acide hyaluronique ou l'acide chlorhydrique.

3. Utilisation selon la revendication 1, dans laquelle l'inhibiteur d'oxydation induite par peroxyde est l'acide citrique ou l'acide ascorbique.

4. Utilisation selon l'une quelconque des revendications 1 à 3, dans laquelle l'excipient est sélectionné dans le groupe constitué par les dérivés de cellulose, les polymères de méthacrylate, les dérivés de polyvinylpyrrolidone, les polysorbates et les polyéthylène glycols.

5. Composition de comprimé à libération prolongée comprenant de la metformine ou un sel pharmaceutiquement acceptable de celle-ci en tant que principe actif unique, au moins un excipient et d'environ 0,01 à environ 10 %, de préférence d'environ 0,05 à environ 5 %, plus préférablement d'environ 0,1 à environ 2 %, d'un inhibiteur d'oxydation induite par peroxyde, par rapport au poids total des excipients, dans laquelle l'inhibiteur d'oxydation induite par peroxyde est un antioxydant ou un acide stabilisant, et dans laquelle la composition n'est par une composition effervescente ou gastroflottante qui comprend un acide conjointement avec une base pour la libération de dioxyde de carbone.

6. Composition selon la revendication 5, dans laquelle
(i) l'antioxydant est un antioxydant hydrosoluble, éventuellement dans laquelle l'antioxydant hydrosoluble est l'acide ascorbique, l'acétyl cystéine, la cystéine, la thiourée, le **1-**thiosorbitol, un sulfite, un bisulfite, un métabisulfite, un thiosulfate ou un sel pharmaceutiquement acceptable de ceux-ci, et/ou
(ii) l'acide stabilisant est l'acide citrique, l'acide tartrique, l'acide phosphorique, l'acide malique, l'acide lactique, l'acide méthanesulfonique, l'acide éthanesulfonique, l'acide*p-*toluènesulfonique, l'acide oxalique, l'acide malonique, l'acide succinique, l'acide glutarique, l'acide adipique, l'acide gluconique, l'acide glucuronique, l'acide glutamique, l'acide fumarique, l'acide maléique, l'acide hyaluronique ou un acide chlorhydrique.

7. Composition selon la revendication 5, dans laquelle l'inhibiteur d'oxydation induite par peroxyde est l'acide citrique ou l'acide ascorbique.

8. Composition selon l'une quelconque des revendications 5 à 7, dans laquelle l'excipient est sélectionné dans le groupe constitué par les dérivés de cellulose, les polymères de méthacrylate, les dérivés de polyvinylpyrrolidone, les polysorbates et les polyéthylène glycols.

9. Composition selon l'une quelconque des revendications 5 à 8, comprenant
(i)
(a) 40 à 90 %, de préférence 50 à 80 %, par exemple 55 à 75 %, par rapport au poids de la composition, de metformine ou d'un sel pharmaceutiquement acceptable de celle-ci,
(b) 0,01 à 5 %, de préférence 0,02 à 2 %, par exemple 0,05 à 1 %, par exemple 0,1 à 0,5 %, par rapport au poids de la composition, d'un inhibiteur d'oxydation induite par peroxyde ;
(c) 5 à 60 %, de préférence 10 à 50 %, par exemple 20 à 40 %, par rapport au poids de la composition, d'au moins un excipient, ou
(ii)
(a) 40 à 90 %, de préférence 50 à 80 %, par exemple 55 à 75 %, par rapport au poids de la composition, de metformine ou d'un sel pharmaceutiquement acceptable de celle-ci,
(b) 0,01 à 5 %, de préférence 0,02 à 2 %, par exemple 0,05 à 1 %, par exemple 0,1 à 0,5 %, par rapport au poids de la composition, d'un inhibiteur d'oxydation induite par peroxyde ;
(c) 0,5 à 10 %, de préférence 1 à 8 %, par exemple 2 à 5 %, par rapport au poids de la composition, d'un liant ;
(d) 5 à 50 %, de préférence 10 à 45 %, par exemple 15 à 40 %, par rapport au poids de la composition, d'un polymère de régulation de libération sélectionné parmi les dérivés de cellulose et les polymères de méthacrylate ; et
(e) 0,1 à 5 %, de préférence 0,2 à 2 %, par exemple 0,3 à 1 %, par rapport au poids de la composition, d'un lubrifiant sélectionné parmi le stéarate de magnésium et l'acide stéarique.

10. Procédé de préparation d'une composition de comprimé à libération prolongée comprenant de la metformine ou un sel pharmaceutiquement acceptable de celle-ci en tant que principe actif unique et au moins un excipient, dans lequel la composition n'est pas une composition effervescente ou gastroflottante qui comprend un acide conjointement avec une base pour la libération de dioxyde de carbone, ledit procédé comprenant la mise en contact de l'au moins un excipient avec une quantité d'un inhibiteur d'oxydation induite par peroxyde efficace pour réduire la formation de nitrosamines dans la composition, dans lequel l'inhibiteur d'oxydation induite par peroxyde est un antioxydant ou un acide stabilisant.

11. Procédé selon la revendication 10, dans lequel
(i) l'antioxydant est un antioxydant hydrosoluble, éventuellement dans lequel l'antioxydant hydrosoluble est l'acide ascorbique, l'acétyl cystéine, la cystéine, la thiourée, le 1-thiosorbitol, un sulfite, un bisulfite, un métabisulfite, un thiosulfate ou un sel pharmaceutiquement acceptable de ceux-ci, et/ou
(ii) l'acide stabilisant est l'acide citrique, l'acide tartrique, l'acide phosphorique, l'acide malique, l'acide lactique, l'acide méthanesulfonique, l'acide éthanesulfonique, l'acide *p-*toluènesulfonique, l'acide oxalique, l'acide malonique, l'acide succinique, l'acide glutarique, l'acide adipique, l'acide gluconique, l'acide glucuronique, l'acide glutamique, l'acide fumarique, l'acide maléique, l'acide hyaluronique ou un acide chlorhydrique.

12. Procédé selon la revendication 10, dans lequel l'inhibiteur d'oxydation induite par peroxyde est l'acide citrique ou l'acide ascorbique.

13. Procédé selon l'une quelconque des revendications 10 à 12, dans lequel l'excipient est sélectionné dans le groupe constitué par les dérivés de cellulose, les polymères de méthacrylate, les dérivés de polyvinylpyrrolidone, les polysorbates et les polyéthylène glycols.

14. Procédé selon l'une quelconque des revendications 10 à 13, comprenant les étapes consistant à
(a) dissoudre l'inhibiteur d'oxydation induite par peroxyde dans un liquide de granulation comprenant de l'eau ;
(b) granuler le mélange comprenant au moins un excipient avec le liquide de granulation de l'étape (a) ;
(c) sécher les granulés humides ;
(d) éventuellement mélanger les granulés avec d'autres excipients ; et
(e) comprimer la masse résultante en comprimés ; et, éventuellement, enrober le comprimé avec un ou un autre agent de pelliculage pharmaceutiquement acceptable.

15. Procédé selon la revendication 14, dans lequel le mélange de l'étape (b) comprend également de la metformine ou un sel pharmaceutiquement acceptable de celle-ci, éventuellement dans lequel l'étape (c) est suivie par une autre étape consistant à mélanger de la metformine ou un sel pharmaceutiquement acceptable de celle-ci avec les granulés obtenus à l'étape (c) avant une autre granulation du mélange résultant avec un liquide de granulation comprenant de l'eau ; et sécher les granulés résultants.
